(19) Europäisches Patentamt
European Patent Office
Office européen des brevets

(11) Veröffentlichungsnummer: **0 268 931**
**B1**

# (12) EUROPÄISCHE PATENTSCHRIFT

(45) Veröffentlichungstag der Patentschrift: **01.08.90**

(51) Int. Cl.[5]: **C07C 67/297**, C07C 69/145

(21) Anmeldenummer: **87116622.9**

(22) Anmeldetag: **11.11.87**

## (54) Verfahren zur Herstellung von 2-sunstituierten 4-Acyloxi-2-butenalen.

(30) Priorität: **20.11.86 DE 3639562**

(43) Veröffentlichungstag der Anmeldung: **01.06.88 Patentblatt 88/22**

(45) Bekanntmachung des Hinweises auf die Patenterteilung: **01.08.90 Patentblatt 90/31**

(84) Benannte Vertragsstaaten: **CH DE FR GB LI**

(56) Entgegenhaltungen:
**EP-A- 0 010 656**
**EP-A- 0 058 927**
**WO-A-79/00485**
**DE-B- 1 297 597**
**FR-A- 2 124 608**

(73) Patentinhaber: **BASF Aktiengesellschaft, Carl-Bosch-Strasse 38, D-6700 Ludwigshafen(DE)**

(72) Erfinder: **Merger, Franz, Dr., Max-Slevogt-Strasse 25, D-6710 Frankenthal(DE)**
Erfinder: **Fischer, Rolf, Dr., Bergstrasse 98, D-6900 Heidelberg(DE)**
Erfinder: **Horler, Hans, Dr., Hainweg 20, D-6100 Darmstadt(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur Herstellung von 2-substituierten 4-Acyloxi-2-butenalen durch Umsetzen von Acyloxiacetaldehyden mit Aldehyden, die 2 H-Atome in α-Stellung aufweisen.

4-Acyloki-2-alkyl-2-butenale sind wertvolle Vausteine für die Herstellung von Terpenen mit biologischer und pharmakologischer Wirksamkeit, wie dem Vitamin A. Dementsprechend ist eine Reihe von Berfahren zu ihrer Herstellung beschrieben worden. So läßt sich beispielsweise (E)-4-Acetoki-2-methyl-2-butenal ausgehend von Butindiol herstellen. Hierzu wird Butindiol zunächst partiell zu 2-Buten-1,4-diol hydriert, das dann zu 1-Buten-3,4-diol umgelagert und schließlich zu 1-Buten-3,4-dioldiacetat acetyliert werden kann. Aus letzterem erhält man durch Hydroformylierung und Abspalten von Essigsäure (E)-4-Acetoxi-2-methyl-2-butenal (vgl. DE 1 941 632).

Eine weitere bekannte Herstellmöglichkeit sieht folgendermaßen aus: Aus Methylglyoxaldimethylacetal wird durch Äthinylierung und anschließende Partialhydrierung 2-Hydroxi-2-methyl-3-butenal-dimethylacetal hergestellt, dieses wird acetyliert und das erhaltene 2-Acetoxi-2-methyl-3-butenal-dimethylacetal in Gegenwart von Kupfer-Katalysatoren zu 4-Acetoxi-2-methyl-2-butenal-dimethylacetal umgelagert. Letzteres kann dann selektiv zu (E)-4-Acetoxi-2-methyl-2-butenal hydrolysiert werden (vgl. DE 1 297 597).

Nachteilig an diesen Synthesewegen ist die große Anzahl von Reaktionsschritten, die zur Herstellung der gewünschten 4-Acyloxi-2-alkyl-2-butenale notwendig sind.

Es war daher die Aufgabe der Erfindung, ein Verfahren zu entwickeln, mit dessen Hilfe die begehrten 4-Acyloxi-2-alkyl-2-butenale auf wesentlich einfachere Weise hergestellt werden können.

Gegenstand der Erfindung ist ein Verfahren zur Herstellung von 2-substituierten 4-Acyloxi-2-butenalen der allgemeinen Formel I

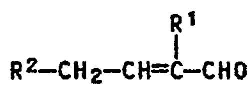 (I),

in der $R^1$ einen Alkylrest mit 1 bis 12 C-Atomen, vorzugsweise 1 bis 4 C-Atomen bedeutet, der durch cycloaliphatische, aromatische oder heterocyclische Reste, einen Alkenyl- oder Alkinylrest oder durch Hydroxi-, Ether-, Thioether-, Acyl-, Alkylamino-, Carboxi- oder Carbalkoxigruppen substituiert sein kann und $R^2$ für eine Acyloxigruppe mit 1 bis 12 C-Atomen, vorzugsweise 1 bis 4 C-Atomen steht, das dadurch gekennzeichnet ist, daß man einen Acyloxiacetaldehyd der allgemeinen Formel II

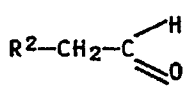 (II),

in der $R^2$ die oben angegebene Bedeutung hat, mit einem Aldehyd der allgemeinen Formal III

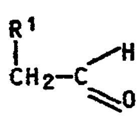 (III),

in der $R^1$ die oben angegebene Bedeutung hat, in Gegenwart eines sekundären Amins und einer organischen Säure bei Temperaturen von 20 bis 100°C, vorzugsweise 30 bis 80°C, umsetzt.

Das vorteilhafte Ergebnis des erfindungsgemäßen Verfahrens, das es erlaubt, Acyloxiaceteldehyde der Formel II in nur einem Reaktionsschritt mit den Aldehyden der Formel III im wesentlichen zu den begehrten 2-substituierten 4-Acyloxi-2-butenalen umzusetzen, konnte aufgrund der oben erläuterten technischen Kenntnisse nicht erwartet werden. Es war überraschend, daß unter den erfindungsgemäßen Reaktionsbedingungen bei dieser Umsetzung von zwei verschiedenen Aldehyden, die beide je zwei reaktionsfähige H-Atome in α-Stellung zur Formylgruppe aufweisen, bevorzugt die begehrten 2-substituierten 4-Acyloxi-2-butenale gebildet werden und nicht eine Vielzahl von Kondensationsprodukten, die hätte erwartet werden können. Von den möglichen Kondensationsprodukten der Formeln

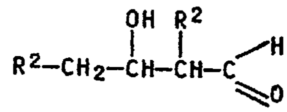

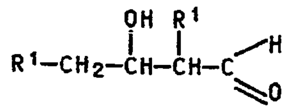

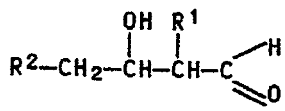

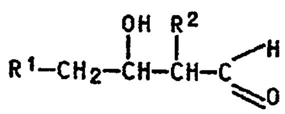

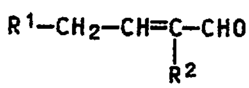

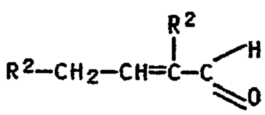

und 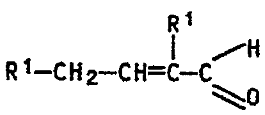

tritt nur das letztgenannte Autokondensationsprodukt des Aldehyds der Formel III als Nebenprodukt in etwas größerer Menge auf.

Als einsetzbare Acyloxiaceteldehyde der Formel II seien beispielsweise genannt: Acetoxi-, Propionyloxi-, Butyryloxi-, Isobutyryloxi- und Pivaloyloxiaceteldehyd. Bevorzugt werden Acetaldehyde der Formel II, in denen $R^2$ für eine Acyloxigruppe mit vorzugsweise 1 bis 4 C-Atomen steht. Besondere Bedeutung hat die Umsetzung mit Acetoxiacetaldehyd.

In den Aldehyden der Formel III bedeutet $R^1$ z.B. einen geradkettigen oder verzweigten Alkylrest mit 1 bis 12, vorzugsweise 1 bis 8 C-Atomen, insbesondere 1 bis 4 C-Atomen, der cycloaliphatische, aromatische oder heterocyclische Reste, wie Phenyl- oder Pyridyl-Reste, einen Alkenyl- oder Alkinylrest und Hydroxi-, Alkoxi-, Thioether-, Acetoxi-, Alkylamino-, Carboxi- oder Carbalkoxigruppen enthalten kann.

Beispielsweise seien die folgenden Aldehyde der Formel III genannt: Propanal, Butanal, Pentanal, 3-Pentenal, 4-Pentenal, 3-Methyl-butanal, 3-Phenyl-propanal, 3-Phenyl-butanal, 3-Anisyl-propanal und -butanal, 3-Pyridyl-propanal, 4-Hydroxi-butanal, 4-Acetoxi-butanal, 5-Formyl-valeriansäure, 5-Formylvaleriansäureester, 4-Dimethylamino-butanal, 3-Methylthio-propanal, 4-Methylthio-butanal, 3,6-Dioxa-heptanal, 3,5-Dimethyl-oct-5-en-1-al, 4-Oxa-pentanal, 4,7-Dioxa-octanal. Bevorzugte Aldehyde der Formel III sind solche, in denen $R^1$ für einen Alkylrest mit vorzugsweise 1 bis 4 C-Atomen steht. Von besonderer Bedeutung ist die Umsetzung mit Propanal.

Das erfindungsgemäße Verfahren wird in Gegenwart eines Katalysatorsystems durchgeführt, das aus einem linearen oder cyclischen sekundären Amin und einer organischen Säure, insbesondere einer Carbonsäure, besteht. Als lineare sekundäre Amine seien beispielsweise Dialkylamine, wie Dimethylamin, Diethylamin, Diisopropylamin, Di-(iso)-butylamin, Methyl-ethyl-amin, Methyl-butylamin, Ethyl-butylamin und Methyl-hydroxiethylamin genannt. Einsetzbare cyclische Amine sind beispielsweise Pyrrolidin, Piperidin und Morpholin.

Als organische Säuren können einbasische, aber auch zwei- und mehrbasische Säuren verwendet werden. Besonders geeignet sind Mono- und Dicarbonsäuren, wie Essigsäure, Propionsäure, (Iso-)-Buttersäure, (Iso-)Valeriansäure, 2-Methyl-buttersäure, Hexansäure, Methylpentansäure, Ethylhexansäure, Isononansäure, Methoxiessigsäure, Pivalinsäure, Methoxi-pivalinsäure, Oxalsäure, Bernsteinsäure, Glutarsäure, Adipinsäure, Hydroxibuttersäure, Apfelsäure und Hydroxipivalinsäure. Beim Einsatz einer Aldehydsäure, wie 5-Formyl-valeriansäure ist die Verwendung einer zusätzlichen Carbonsäure nicht erforderlich.

Katalysatoren dieser Art werden z.B. in der EP-B-58 927 beschrieben. Bei diesen Katalysatoren ist es nicht erforderlich, aber vorteilhaft, daß die Amine und die Säuren in äquivalenten Mengenverhältnissen eingesetzt werden. Es können jedoch auch 1 bis 1,5 Aquivalente Säure je Äquivalent Amin eingesetzt werden. Die zur Umsetzung eingesetzte Katalysatormenge ist ebenfalls in weiten Grenzen variabel. Zweckmäßigerweise verwendet man eine Katalysatormenge von 1 bis 150, vorzugsweise 5 bis 100, insbesondere 20 bis 100 Mol%, bezogen auf den Ausgangsstoff der Formel II. Setzt man zum Zwecke einer raschen Reaktion under milden Bedingungen mehr als vernachlässigbare "katalytische" Mengen Katalysator (das sind z.B. Katalysatormengen von mehr als 5 Mol%, bezogen auf II) ein, so wird man den Katalysator nach Abtrennung des Produktes, z.B. durch Phasentrennung, Extraktion oder Destillation, zurückgewinnen und wiederholt einsetzen, so daß insgesamt ein geringer Verbrauch resultiert.

Mit besonderem Vorteil gelingt die erfindungsgemäße Kondensation, wenn man sie zusätzlich in Gegenwart von Wasser durchführt, hierbei arbeitet man dann mit etwa 5 bis 70, vorzugsweise etwa 10 bis 50 Gew.% an Wasser im Reaktionsgemisch. In Gegenwart von Wasser ist insbesondere die Aufarbeitung des Reaktionsgemisches leichter, da sich der Katalysator mit Wasser besser abtrennen läßt.

Die Umsetzung wird im allgemeinen bei Temperaturen von etwa 20 bis 100°C, vorzugsweise etwa 30 bis 80°C, durchführt. Im allgemeinen arbeitet man unter Atmosphärendruck; aber auch ein Arbeiten unter vermindertem oder erhöhtem Druck ist prinzipiell möglich. Man kann diskontinuierlich, aber auch kontinuierlich arbeiten.

Die Ausgangsstoffe setzt man in Molverhältnissen von Aldehyd der Formel III : Acetaldehyd der Formel II = 1 : 1 bis 10 : 1, vorzugsweise 1,4 bis 7,5 : 1, insbesondere 1,5 bis 6 : 1 ein.

Zur Durchführung des Verfahrens geht man mit Vorteil so vor, daß man die Ausgangsaldehyde unter Rühren gleichzeitig oder sogar ineinander gelöst langsam in das auf die Reaktionstemperatur erwärmte, gegebenenfalls Wasser enthaltende Katalysatorgemisch einträgt, hierbei durch Kühlung die Reaktionstemperatur beibehält und anschließend das Reaktionsgemisch noch für eine gewisse Zeit zweckmäßig unter Rühren bei der Reaktionstemperatur nachreagieren läßt. Die Dauer der Nachreaktionszeit hängt von der Art der Ausgangsverbindungen sowie von der Reaktionstemperatur ab. Im allgemeinen reicht eine Nachreaktionszeit von 5 bis 240 Minuten aus.

Das erfindungsgemäße Verfahren zeichnet sich also durch eine verfahrenstechnisch einfache Durchführbarkeit aus. Viele der Ausgangsverbindungen sind wirtschaftlich günstig zugänglich. So ist zum Beispiel der für die Herstellung des als Vitamin-A-Zwischenprodukt interessanten 4-Acetoxi-2-methyl-2-butenals benötigte Acetoxiaceteldehyd auf relativ einfache Weise aus Ethylenoxid und Acetanhydrid über die Oxidation von Ethylenglykolmonoacetat zugänglich. Propionaldehyd ist eine handelsübliche Verbindung.

Die nachstehenden Beispiele sollen das erfindungsgemäße Verfahren erläutern.

Beispiele 1 bis 5

In ein aus 120 g (2 mol) Essigsäure und 225 g einer 40gew.-%igen wäßrigen Lösung von Dimethylamin (≠ 2 mol Dimethylamin und 66 Gew.%, bezogen auf eingesetztes II bzw. in Beispielen 1 bis 3 17,3 %, bezogen auf das gesamte Reaktionsgemisch an Wasser) wurde unter Rühren und Kühlen bei der aus der Tabelle 1 ersichtlichen Temperatur (T) und innerhalb der aus der Tabelle 1 ersichtlichen Zeit (Z) eine Lösung der aus Tabelle 1 ersichtlichen Menge an Acetoxiacetaldehyd in der aus der Tabelle 1 ersichtlichen Menge an Propionaldehyd zugefügt. Anschließend wurde das Reaktionsgemisch noch unter Rühren für die aus Tabelle 1 ersichtliche Nachreaktionszeit (NZ) bei der Reaktionstemperatur gehalten. Nach dem Abkühlen des Reaktionsgemisches auf Raumtemperatur wurde die sich als untere Phase absetzende wäßrige Katalysatorlösung abgetrennt und die organische Phase fraktioniert destilliert. Die angegebenen Ausbeuteangaben beziehen sich auf eingesetzten Acetoxiacetaldehyd.

Tabelle 1

| Beispiel | T [°C] | Z [Min.] | Acetoxi-acetaldehyd [g (mol)] | Propion-aldehyd [g (mol)] | NZ [Min.] | (E)-4-Acetoxi-2-methyl-2-butenal Ausbeute [g (% d. Theorie)] | Kp. [°C/mbar] | Ausbeute [g] |
|---|---|---|---|---|---|---|---|---|
| 1 | 50 | 30 | 204 (2) | 232 (4) | 30 | 153 (54) | 60–67/1 | 139 |
| 2 | 80 | 5 | 204 (2) | 232 (4) | 10 | 176 (62) | 60–64/1 | 130 |
| 3 | 30 | 15 | 204 (2) | 232 (4) | 240 | 125 (44) | 58–67/1 | 148 |
| 4 | 50 | 15 | 204 (2) | 128 (2,2) | 30 | 119 (42) | 62–67/1 | 61 |
| 5 | 50 | 30 | 204 (2) | 580 (10) | 30 | 179 (63) | 60–75/2 | 421 |

Beispiel 6

In ein Gemisch aus 225 g einer 40gew.-%igen wäßrigen Lösung von Dimethylamin und 120 g Essigsäure wurde unter Rühren und Kühlen innerhalb von 15 Min. eine Lösung von 204 g Acetoxiacetaldehyd in 288 g (4 mol) n-Butyraldehyd einlaufen lassen und das Reaktionsgemisch anscghließend noch 30 Min. bei 50°C gerührt. Nach Abkühlenlassen der Reaktionslösung und Abtrennen der wäßrigen Katalysatorlösung erhielt man durch fraktionierte Destillation 169 g 4-Acetoxi-2-ethyl-2-butenal (Kp = 83 - 93°C bei 6 mbar), entsprechend einer Ausbeute von 54 % der Theorie, bezogen auf Acetoxiacetaldehyd sowie 181 g 2-Ethyl-2-hexenal (Kp = 38 - 70°C bei 20 - 60 mbar).

**Patentansprüche**

1. Verfahren zur Herstellung von 2-substituierten 4-Acyloxi-2-butenalen der allgemeinen Formel I

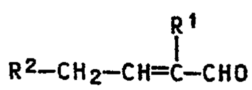 (I),

in der $R^1$ einen Alkylrest mit 1 bis 12 C-Atomen bedeutet, der durch cycloaliphatische, aromatische oder heterocyclische Reste, einen Alkenyl- oder Alkinylrest oder durch Hydroxi-, Ether-, Thioether-, Acyl-, Alkylamino-, Carboxi- oder Carbalkoxigruppen substituiert sein kann und $R^2$ für eine Acyloxigruppe mit 1 bis 12 C-Atomen steht, dadurch gekennzeichnet, daß man einen Acyloxiacetaldehyd der allgemeinen Formel II

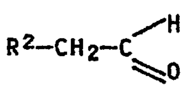 (II),

in der $R^2$ die oben angegebene Bedeutung hat, mit einem Aldehyd der allgemeinen Formel III

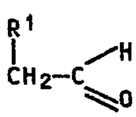 (III),

in der $R^1$ die oben angegebene Bedeutung hat, in Gegenwart eines linearen oder cyclischen sekundären Amins und einer organischen Säure bei Temperaturen von 20 bis 100°C umsetzt.

2. Verfahren gemäß Anspruch 1, dadurch gekennzeichnet, daß man die Aldehyde der Formeln II und III bei Temperaturen von 30 bis 80°C umsetzt.

3. Verfahren gemäß Anspruch 1, dadurch gekennzeichnet, daß man die Umsetzung in Gegenwart einer Mono- oder Di-Carbonsäure mit 1 bis 10 C-Atomen verwendet.

4. Verfahren gemäß Anspruch 1, dadurch gekennzeichnet, daß man als monosubstituierten Acetaldehyd der allgemeinen Formel II Acetoxiacetaldehyd verwendet.

5. Verfahren gemäß Anspruch 1, dadurch gekennzeichnet, daß man als Aldehyd der Formel III Propanal verwendet.

6. Verfahren gemäß Anspruch 1, dadurch gekennzeichnet, daß man die Umsetzung zusätzlich in Gegenwart von Wasser durchführt.

**Claims**

1. A process for preparing a 2-substituted 4-acyloxy-2-butenal of the general formula I

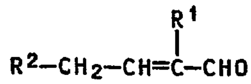

(I),

where $R^1$ is alkyl of 1 to 12 carbon atoms, which may be substituted by cycloaliphatic, aromatic or heterocyclic radicals, by alkenyl or alkynyl or by hydroxyl, ether, thioether, acyl, alkylamino, carboxyl or carbalkoxy, and $R^2$ is acyloxy of 1 to 12 carbone atoms, which comprises reacting an acyloxyacetaldehyde of the general formula II

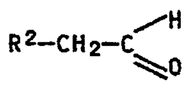

(II),

where $R^2$ has the abovementioned meaning, with an aldehyde of the general formula III

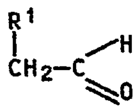

(III),

where $R^1$ has the abovementioned meaning, in the presence of a linear or cyclic secondary amine and of an organic acid at from 20 to 100°C.

2. A process as claimed in claim 1, wherein the aldehydes of the formula II and III are reacted at from 30 to 80°C.

3. A process as claimed in claim 1, wherein the reaction is carried out in the presence of a mono- or dicarboxylic acaid of 1 to 10 carbon atoms.

4. A process as claimed in claim 1, wherein the monosubstituted actealdehyde of the general formula II is acetoxyacetaldehyde.

5. A process as claimed in claim 1, wherein the aldehyde of the formula III is propanal.

6. A process as claimed in claim 1, wherein the reaction is additionally carried out in the presence of water.

**Revendications**

1. Procédé de préparation de 4-acyloxy-2-buténals 2-substitués de formule générale I

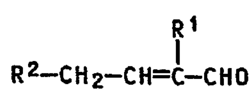

(I),

dans laquelle $R^1$ représente un reste alkyle ayant 1 à 12 atomes de carbone, qui peut être substitué par des restes cycloaliphatiques, aromatiques ou hétérocycliques, un reste alcényle ou alcynyle ou par des groupements hydroxy, éther, thioéther, acyle, alkylamino, carboyx ou carbalcoxy, et $R^2$ est mis pour un groupement acyloxy ayant de 1 à 12 atomes de carbone, caractérisé en ce qu'on fait réagir à des températures de 20 à 100°C un acyloxyacétaldéhyde de formule générale II

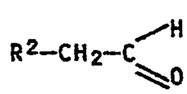

(II),

dans laquelle $R^2$ a la signification donnée ci-dessus, avec un aldéhyde de formule générale III

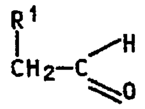 (III),

dans laquelle $R^1$ a la signification donnée ci-dessus, en présence d'une amine secondaire linéaire ou cyclique et d'un acide organique.

2. Procédé selon la revendication 1, caractérisé en ce qu'on fait réagir les aldéhydes de formules II et III à des températures de 30 à 80°C.

3. Procédé selon la revendication 1, caractérisé en ce qu'on effectue la réaction en présence d'un mono- ou diacide carboxylique ayant de 1 à 10 atomes de carbone.

4. Procédé selon la revendication 1, caractérisé en ce qu'on utilise de l'acétoxyacétaldéhyde comme aldéhyde monosubstitué de formule générale II.

5. Procédé selon la revendication 1, caractérisé en ce qu'on utilise de propanal comme aldéhyde de formule III.

6. Procédé selon la revendication 1, caractérisé en ce qu'on effectue la réaction également en présence d'eau.